# EUROPEAN PATENT SPECIFICATION

(11) **EP 2 032 692 B1**
(45) Date of publication and mention of the grant of the patent: **16.11.2016**
(21) Application number: 07812333.8
(22) Date of filing: 26.06.2007
(51) Int. Cl.: C12N 5/0775, C12N 5/077

(54) **METHOD OF CULTURING MESENCHYMAL STEM CELLS**
VERFAHREN ZUR KULTIVIERUNG MESENCHYMALER STAMMZELLEN
PROCÉDÉ DE CULTURE DE CELLULES SOUCHES MÉSENCHYMATEUSES

(30) Priority: 26.06.2006 US 805801 P
(43) Date of publication of application: 11.03.2009
(73) Proprietor: Terumo BCT, Inc., Lakewood, CO 80215 (US)
(72) Inventor: ANTWILER, Glen, Delbert, Lakewood, Colorado 80215 (US)
(74) Representative: Simons, Amanda Louise
(86) International application number: PCT/US2007/072125
(87) International publication number: WO 2008/002914

(56) References cited:
- EP-A2- 1 367 119
- WO-A-96/39487
- WO-A-03/070922
- WO-A-2006/019357
- WO-A2-2005/007799
- COLTER DAVID C ET AL: "Rapid expansion of recycling stem cells in cultures of plastic-adherent cells from human bone marrow" PROCEEDINGS OF THE NATIONAL ACADEMY OF SCIENCES OF USA, NATIONAL ACADEMY OF SCIENCE, WASHINGTON, DC, US, vol. 97, no. 7, 28 March 2000 (2000-03-28), pages 3213-3218, XP002205331 ISSN: 0027-8424
- SEKIYA ICHIRO ET AL: "Expansion of human adult stem cells from bone marrow stroma: Conditions that maximize the yields of early progenitors and evaluate their quality." STEM CELLS (MIAMISBURG), vol. 20, no. 6, 2002, pages 530-541, XP009092467 ISSN: 1066-5099
- SOTIROPOULOU PANAGIOTA A ET AL: "Characterization of the optimal culture conditions for clinical scale production of human mesenchymal stem cells" STEM CELLS (MIAMISBURG), vol. 24, no. 2, February 2006 (2006-02), pages 462-471, XP009066391 ISSN: 1066-5099
- JAVAZON ELISABETH H ET AL: "Rat marrow stromal cells are more sensitive to plating density and expand more rapidly from single-cell-derived colonies than human marrow stromal cells" STEM CELLS (MIAMISBURG), vol. 19, no. 3, 2001, pages 219-225, XP009092481 ISSN: 1066-5099
- CAMPAGNOLI C ET AL: "Identification of mesenchymal stem/progenitor cells in human first-trimester fetal blood, liver, and bone marrow", BLOOD, AMERICAN SOCIETY OF HEMATOLOGY, US, vol. 98, no. 8, 15 October 2001 (2001-10-15), pages 2396-2402, XP002328674, ISSN: 0006-4971, DOI: 10.1182/BLOOD.V98.8.2396
- DE KREUK A M ET AL: "A single-step colony-forming unit assay for unseparated mobilized peripheral blood, cord blood, and bone marrow.", JOURNAL OF HEMATOTHERAPY & STEM CELL RESEARCH DEC 2001, vol. 10, no. 6, December 2001 (2001-12), pages 795-806, XP009179343, ISSN: 1525-8165
- DEANS ROBERT J ET AL: "Mesenchymal stem cells: Biology and potential clinical uses", EXPERIMENTAL HEMATOLOGY, ELSEVIER INC, US, vol. 28, no. 8, 1 August 2000 (2000-08-01) , pages 875-884, XP002201188, ISSN: 0301-472X, DOI: 10.1016/S0301-472X(00)00482-3

## Description

### PRIORITY CLAIM

This application claims priority from United States Provisional patent application No. 60/805,801 filed June 26, 2006.

### BACKGROUND

Human stem cells, which have been expanded in culture from a small amount of donor cells, can be used to repair or replace damaged or defective tissues and have broad clinical applications for treatment of a wide range of diseases. Recent advances in the area of regenerative medicine demonstrates that stem cells have unique properties such as self-renewal capacity, the ability to maintain the unspecialized state, and the ability to differentiate into specialized cells under particular conditions.

As an important component of regenerative medicine, the bioreactor or cell expansion system plays an important role in providing optimized environments for cell growth and expansion. The bioreactor provides nutrients to the cells and removal of metabolites, as well as furnishing a physiochemical environment conducive to cell growth in a closed, sterile system.

Many types of bioreactors are currently available. Two of the most common include flat plate bioreactors and hollow fiber bioreactors. Flat plate bioreactors enable cells to grow on large flat surfaces, while hollow fiber bioreactors enable cells to grow either on the inside or outside of the hollow fibers.

Conventional wisdom in the area of stem cell growth and replication has held that in a bioreactor or other ex vivo cell expansion system, adherent cells such as mesenchymal stem cells (MSCs) replicate faster when they are closely surrounded by other MSCs, and therefore a high number of MSCs should be initially loaded into a cell expansion system. Conventional wisdom also has held that the best MSCs for expansion in a bioreactor are those cells which have been highly purified or separated away from other contaminating cell types before being expanded in a bioreactor. The method of this invention teaches that surprisingly, this is not the case.

### BRIEF DESCRIPTION OF THE FIGURES

FIG. 1 is a schematic view of a bioreactor which may be used in this invention.
FIG. 2 is a graph comparing the growth of mesenchymal stem cells having different levels of purity 7 days after reseeding.
FIG. 3 is a graph comparing the growth of mesenchymal stem cells having different levels of purity 14 days after reseeding.
FIG. 4 is another graph comparing the growth of mesenchymal stem cells having different levels of purity.

### SUMMARY OF THE INVENTION

This invention is directed toward a method of expanding human mesenchymal stem cells ex vivo including the steps of directly seeding a collection of unpurified cells containing the mesenchymal stem cells on a substrate so that a low density of mesenchymal stem cells adhere to the substrate, and expanding the adhered mesenchymal stem cells on the substrate. Optionally, the expanded mesenchymal stem cells may be removed from the substrate. Optionally, the mesenchymal stem cells may be reseeded on the same or different substrate.

### DETAILED DESCRIPTION

This invention relates to methods of seeding and expanding MSCs in a cell culturing system.

As discussed above, a number of bioreactor configurations exist for culturing anchorage-dependent cells such as MSCs, and this invention is not dependent upon any particular configuration.

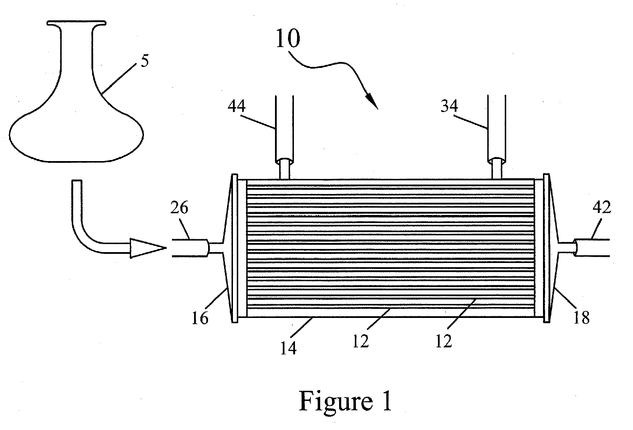

However, as but one example, not meant to be limiting, is a hollow fiber bioreactor shown in FIG. 1. A cell expansion module or bioreactor **10** which may be used in the present invention is made of a bundle of hollow fiber membranes **12** enclosed within a housing **14.** The bundle of hollow fibers is collectively referred to as a membrane. The housing or module **14** may be cylindrical in shape and may be made of any type of biocompatible polymeric material.

Each end of the module **14** is closed off with end caps or headers **16, 18.** The end caps **16, 18** may be made of any suitable material such as polycarbonate so long as the material is biocompatible with the cells to be grown in the bioreactor.

There may be at least four ports into and out of the module. Two ports fluidly connect to the extracapillary space (EC space), one port **34** for fresh extracapillary media ingress into the space surrounding the hollow fibers and one port **44** for spent extracapillary media egress out of the module. Two ports also fluidly connect to the intracapillary space (IC space), one port **26** for fresh intracapillary media ingress into the lumen of the hollow fibers as well as for the introduction of the cells to be expanded, and one port **42** for spent intracapillary media egress and removal of expanded cells from the bioreactor.

Cells to be expanded in the bioreactor may be flowed into the IC space or the EC space. The bioreactor may be loaded with cells using a syringe or the cells may be distributed into the IC or EC spaces directly from a cell separator. The cells may also be introduced into the growth module or bioreactor from a cell input bag or flask (shown as element **5**) which may be sterile docked to the bioreactor.

As discussed above, the number of cells initially seeded in a cell expansion system is thought to be an important factor in how quickly cells expand and reach confluency in a cell expansion system such as the one described above. However, Example 1 below shows that in fact, MSCs do not have to be closely surrounded by other MSCs to achieve optimum growth and expansion.

### Example 1

MSCs were initially seeded in flasks at concentrations of 5, 50, 500 and 5000 cells/cm². The time (in hours) it took the cells to double was measured. As shown in Table 1 below, the average doubling time for cells at an initial concentration of 5 cells/cm² is around 35 hours. Cells at a thousand-fold higher concentration took around 123 hours to double. This data suggests that cells initially seeded at lower densities double more quickly than cells initially seeded at higher densities. Lower density is defined as between around 1-100 cells/cm² on a substrate, including between around 5-50 cells/cm² on a substrate.

**Table 1**

| **Seeding density MSCs/cm²)** | **Doubling time (h)** |
|---|---|
| 5000 | 123 |
| 500 | 56 |
| 50 | 42 |
| 5 | 35 |

Another factor thought to be important in mesenchymal cell expansion is the purity of the cells initially loaded or seeded in a cell expansion system.

### Example 2

To test the theory that to insure maximal expansion of MSCs, MSCs must be purified from other contaminating cells, a four-armed experiment was performed. For the purposes of this and other examples, a purification procedure means any additional procedures performed to remove substantially all contaminating cells from a collection of cells which contain MSCs. The collection of cells could be cells from bone marrow, peripheral blood, umbilical cord, embryonic or any other collection of cells where MSCs reside. Unpurified cells mean any of the above cells not subjected to a purification procedure.

For each arm, after the purification and fractionation step (if performed) the cells were counted. The same number of cells purified (or not) from each arm were plated. The cells were grown until around 1 x 10⁶ cells were obtained (confluency for the purposes of this example). Confluency is defined as the state at which the cells have expanded so that they cover the entire available surface of the substrate they are being grown on.

For each arm, two sets of experiments were done, one set involved passaging or reseeding the cells every 7 days, and the other set involved passaging or reseeding the cells every 14 days. When the cells reached confluency, the experiments were stopped.

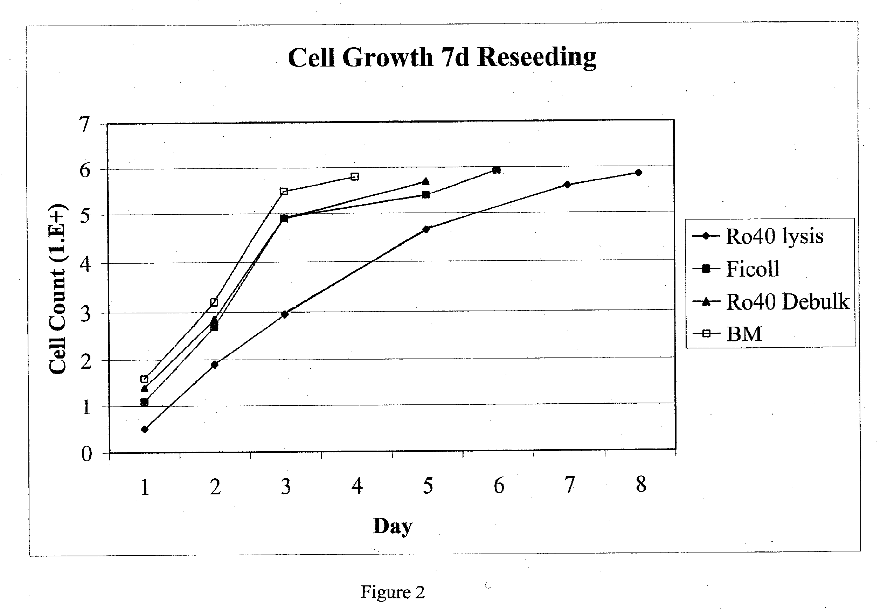

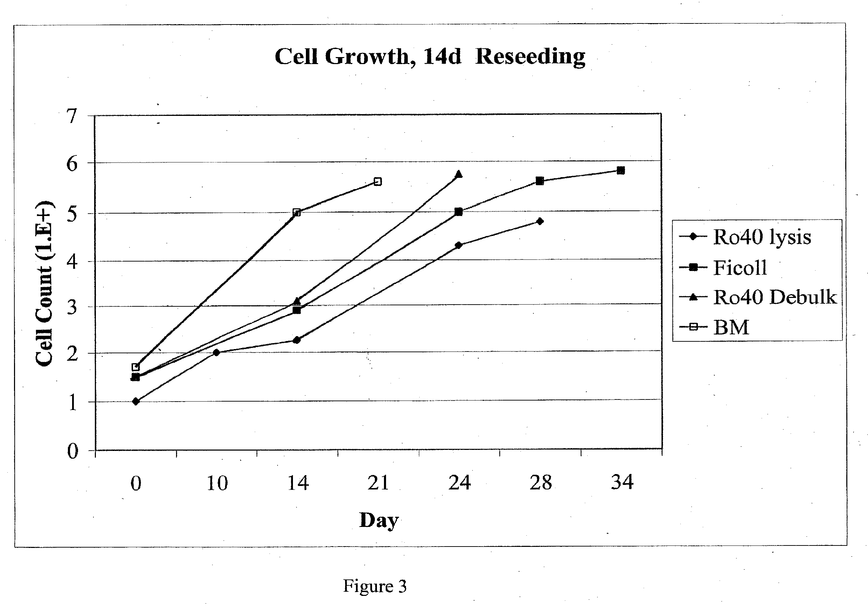

In arm 1, around 50 mL of bone marrow aspirate was removed from a donor and 1 mL was directly plated into a 25 cm² T-flask (designated as BM in FIGS. 2 and 3). No purification or fractionation step was performed. The surface area of a 25 cm² T-flask approximates the surface area of a 1.6 m² hollow fiber bioreactor.

In arm 2, 10 mL of the 50 mL bone marrow aspirate collected from arm 1 was purified to collect the mononuclear cell (MNC) fraction (where stem cells are found) using standard ficoll gradient purification (designated as Ficoll in FIGS. 2 and 3). The fraction was plated onto 25 cm² T-flasks.

Arm 3 involved elutriating MSCs from the remaining 40 mL bone marrow aspirate collected in arm 1. MSCs were debulked of contaminating red blood cells and elutriated using the Elutra system (available from Gambro BCT, Inc., Lakewood, CO, USA) (designated as Ro40 Debulk in FIGS. 2 and 3).

In arm 4, the remaining unplated fraction from arm 3 was subjected to a lysing procedure to remove any remaining contaminating red blood cells (designated as Ro40 lysis in FIGS. 2 and 3).

Representative graphs are shown in FIGS. 2 and 3. As can be seen, the mesenchymal stem cells which were reseeded every 7 days (FIG. 2) reached confluency at a faster rate then the cells which were reseeded every 14 days (FIG. 3). Surprisingly, the cells which received the least amount of manipulation (directly plated bone marrow containing contaminating cells) were the fastest growing. Cells which received the greatest manipulation, and thus were assumed to have the greatest purity, (elutriated MSCs with any contaminating red blood cells lysed) were the slowest growing.

These findings also suggest that MSCs which were reseeded before reaching confluency grow at least as fast as the MSCs which were seeded initially after collection. This finding might imply that the reseeding process has no adverse effect on MSC growth in a bioreactor, and that reusing (reseeding) the same surface the cells were initially seeded on appears to have no adverse effect on the growth of the cells. However, the cells could be reseeded on a different substrate as well.

It is therefore desirable not to grow MSCs to confluency. Cells should be harvested well before a confluent state is reached. The growth of enough cells to provide a sufficient therapeutic dose of MSCs would be reached quickly.

### Example 3

In another example, a standard elutriation protocol such as that described in US Patent Application 11/131063 was used to enrich subsets of stem cells from bone marrow to determine how cells which were more purified would grow as compared to cells which were not purified away from contaminating cells.

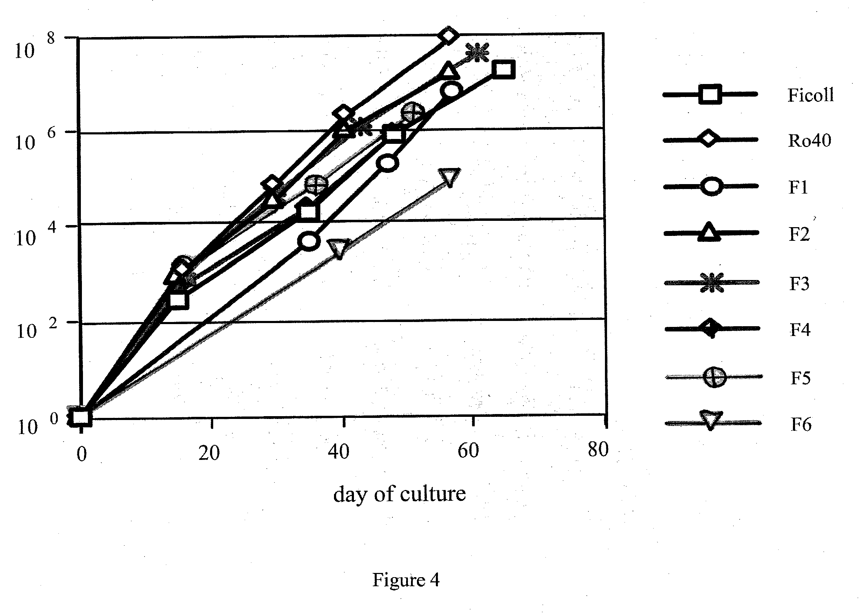

Six elutriated fractions of bone marrow containing substantially purified MSCs were grown to confluency. Substantially purified means that the majority of cells in a particular elutriated fraction are the same cell type. These elutriated fractions are shown as F1-F6 in FIG. 4. A 50 mL sample of bone marrow was also purified using the commonly used purification procedure of a Ficoll gradient for comparison (shown as Ficoll in FIG. 4). 40 rotor off (shown as Ro40 in FIG. 4) means that after the plasma, platelets and red blood cells were removed from the initial 50 mL bone marrow sample, the rotor was turned off and all the cells remaining in the 40 mL elutriation chamber were collected. This procedure does not elute individual fractions or subsets of cells.

FIG. 4 compares the time in days for the cells from the individual elutriated fractions as well as the ficoll purified cells and the cells collected with the rotor off to expand to 1 x 10⁸ cells. As can be seen, fractions F1 - F6 containing the most substantially purified cells grew the slowest, while the least purified cells (rotor off or Ro40) grew the fastest. This corroborates the findings in Example 2 above, that cells which are the least purified, grow the fastest.

### Example 4

As discussed above, the purity of the MSCs to be expanded is thought to effect how quickly the cells expand in culture.

Example 4 compares the alpha (α), or time in days it takes for cells to double, of unpurified MSCs in 50 mLs of bone marrow which was directly seeded into a hollow fiber bioreactor, with MSCs which were pre-selected before being seeded into the bioreactor. Direct seeding means that bone marrow taken from a patient was put directly into a bioreactor without removing any contaminating cells such as platelets and red blood cells. Bone chips and other non-cellular matter may be removed. Pre-selected means that 50 mLs of bone marrow was initially plated on a T-75 flask and the MSCs contained therein were allowed to attach to the flask and grow to confluency before being reseeded in a hollow fiber bioreactor. Pre-selection ensures that all of the cells to be expanded in the bioreactor are pure MSCs. Two types of hollow fiber membranes, a 0.5% thermoplastic polyurethane sold under the trade name Desmopan® (available from Bayer MaterialScience AG, DE) and Polyflux® (a blend of polyamide, polyarylethersulfone and polyvinylpyrrolidone) (available from Gambro Dialysatoren, GmBH, Hechingen, DE) were tested. Both Desmopan and Polyflux are synthetic membranes.

**Table 2**

| | **Direct seeded bioreactor** | **Bioreactor with pre-selected MSCs** |
|---|---|---|
| | **α** | **α** |
| **0.5% Desmopan** | 17.3 | 26.7 |
| **Polyflux** | 16.8 | 23.7 |

As is seen from Table 2 above, MSCs which were directly seeded into a bioreactor with other contaminating cells in the bone marrow doubled at a faster rate (took less days to double) than MSCs which were purified first before being expanded in a bioreactor. This is true for both types of synthetic membranes.

As shown in the above examples, to achieve faster MSC expansion in a cell expansion system, the cells need not be purified away from other contaminating cells. MSCs should also be reseeded often and not be allowed to reach confluency before being reseeded.

## Claims

1. A method of expanding human mesenchymal stem cells ex vivo in a hollow fiber bioreactor, the method comprising the steps of:
a.) seeding a collection of unpurified cells containing the mesenchymal stem cells at a density of 1 - 100 mesenchymal stem cells/cm² into the hollow fiber bioreactor so that the mesenchymal stem cells adhere to a hollow fiber membrane, the collection of cells being bone marrow cells, peripheral blood cells, or umbilical cord cells which have not been subject to a purification procedure; and
b.) expanding the adhered mesenchymal stem cells on the hollow fiber membrane.

2. The method of claim 1, wherein the step of seeding comprises seeding unpurified bone marrow cells containing the mesenchymal stem cells.

3. The method of claim 1, wherein the step of seeding comprises seeding unpurified peripheral blood cells containing the mesenchymal stem cells.

4. The method of claim 1, wherein the step of seeding comprises seeding unpurified umbilical cord cells containing the mesenchymal stem cells.

5. The method of claim 1, wherein the hollow fiber membrane comprises a synthetic membrane.

6. The method of claim 1, further comprising the step of removing the expanded mesenchymal stem cells from the hollow fiber bioreactor.

7. The method of claim 6, further comprising the step of reseeding the removed expanded mesenchymal stem cells on the same hollow fiber membrane until a desired number of mesenchymal stem cells is reached.

8. The method of claim 6, further comprising the step of reseeding the removed expanded mesenchymal stem cells on a different hollow fiber membrane until a desired number of mesenchymal stem cells is reached.

9. The method of claim 1, wherein the density at which the mesenchymal stem cells are directly seeded is between around 5-50 mesenchymal stem cells/cm².

10. The method of claim 1, wherein the density at which the mesenchymal stem cells are directly seeded comprises a density of around 5 mesenchymal stem cells/cm².

11. The method of claim 1, wherein the density at which the mesenchymal stem cells are directly seeded comprises a density of around 50 mesenchymal stem cells/cm².

12. The method of claim 7 or claim 8, wherein the step of reseeding the mesenchymal stem cells further includes reseeding the mesenchymal stem cells before they reach confluency.

## Patentansprüche

1. Verfahren zum Erweitern menschlicher mesenchymaler Stammzellen ex vivo in einem Hohlfaser-Bioreaktor, wobei das Verfahren die folgenden Schritte umfasst:
a) Aussäen einer Sammlung ungereinigter Zellen, die die mesenchymalen Stammzellen enthalten, bei einer Dichte von 1 - 100 mesenchymalen Stammzellen/cm² in den Hohlfaser-Bioreaktor, so dass die mesenchymalen Stammzellen an einer Hohlfasermembran anhaften, wobei die Sammlung der Zellen Knochenmarkzellen, periphere Blutzellen oder Nabelschnurzellen sind, die keinem Reinigungsverfahren unterzogen wurden; und
b) Erweitern der angehafteten mesenchymalen Stammzellen auf der Hohlfasermembran.

2. Verfahren nach Anspruch 1, wobei der Schritt des Aussäens das Aussäen ungereinigter Knochenmarkzellen umfasst, die die mesenchymalen Stammzellen enthalten.

3. Verfahren nach Anspruch 1, wobei der Schritt des Aussäens das Aussäen ungereinigter peripherer Blutzellen umfasst, die die mesenchymalen Stammzellen enthalten.

4. Verfahren nach Anspruch 1, wobei der Schritt des Aussäens das Aussäen ungereinigter Nabelschnurzellen umfasst, die die die die mesenchymalen Stammzellen enthalten.

5. Verfahren nach Anspruch 1, wobei die Hohlfasermembran eine synthetische Membran umfasst.

6. Verfahren nach Anspruch 1, ferner umfassend den Schritt des Entfernens der erweiterten mesenchymalen Stammzellen aus dem Hohlfaser-Bioreaktor.

7. Verfahren nach Anspruch 6, ferner umfassend den Schritt des erneuten Aussäens der entfernten erweiterten mesenchymalen Stammzellen auf derselben Hohlfasermembran bis eine gewünschte Anzahl an mesenchymalen Stammzellen erreicht wurde.

8. Verfahren nach Anspruch 6, ferner umfassend den Schritt des erneuten Aussäens der entfernten erweiterten mesenchymalen Stammzellen auf einer anderen Hohlfasermembran bis eine gewünschte Anzahl an mesenchymalen Stammzellen erreicht wurde.

9. Verfahren nach Anspruch 1, wobei die Dichte, zu der die mesenchymalen Stammzellen direkt ausgesät werden, zwischen ca. 5 - 50 mesenchymalen Stammzellen/cm² beträgt.

10. Verfahren nach Anspruch 1, wobei die Dichte, in der die mesenchymalen Stammzellen direkt ausgesät werden, eine Dichte von ca. 5 mesenchymalen Stammzellen/cm² umfasst.

11. Verfahren nach Anspruch 1, wobei die Dichte, in der die mesenchymalen Stammzellen direkt ausgesät werden, eine Dichte von ca. 50 mesenchymalen Stammzellen/cm² umfasst.

12. Verfahren nach Anspruch 7 oder 8, wobei der Schritt des erneuten Aussäens der mesenchymalen Stammzellen ferner das erneute Aussäen der mesenchymalen Stammzellen beinhaltet, bevor sie eine Konfluenz erreichen.

## Revendications

1. Procédé de développement de cellules souches mésenchymateuses humaines ex vivo dans un bioréacteur à fibres creuses, le procédé comprenant les étapes de :
a) l'ensemencement d'une collection de cellules non purifiées contenant les cellules souches mésenchymateuses à une densité de 1 - 100 cellules souches mésenchymateuses/cm² dans le bioréacteur à fibres creuses pour que les cellules souches mésenchymateuses adhèrent à une membrane à fibres creuses, la collection de cellules étant des cellules de moelle osseuse, des cellules de sang périphérique, ou des cellules de cordon ombilical qui n'ont pas été soumises à une procédure de purification ; et
b) le développement des cellules souches mésenchymateuses adhérées, sur la membrane à fibres creuses.

2. Procédé selon la revendication 1, dans lequel l'étape de l'ensemencement comprend l'ensemencement de cellules de moelle osseuse non purifiées contenant les cellules souches mésenchymateuses.

3. Procédé selon la revendication 1, dans lequel l'étape de l'ensemencement comprend l'ensemencement de cellules de sang périphérique non purifiées contenant les cellules souches mésenchymateuses.

4. Procédé selon la revendication 1, dans lequel l'étape de l'ensemencement comprend l'ensemencement de cellules de cordon ombilical non purifiées contenant les cellules souches mésenchymateuses.

5. Procédé selon la revendication 1, dans lequel la membrane à fibres creuses comprend une membrane synthétique.

6. Procédé selon la revendication 1, comprenant en outre l'étape de l'enlèvement des cellules souches mésenchymateuses développées à partir du bioréacteur à fibres creuses.

7. Procédé selon la revendication 6, comprenant en outre l'étape du réensemencement des cellules souches mésenchymateuses développées enlevées sur la même membrane à fibres creuses jusqu'à ce qu'un nombre souhaité de cellules souches mésenchymateuses soit atteint.

8. Procédé selon la revendication 6, comprenant en outre l'étape du réensemencement des cellules souches mésenchymateuses développées enlevées sur une membrane à fibres creuses différente jusqu'à ce qu'un nombre souhaité de cellules souches mésenchymateuses soit atteint.

9. Procédé selon la revendication 1, dans lequel la densité à laquelle les cellules souches mésenchymateuses sont directement ensemencées est entre environ 5-50 cellules souches mésenchymateuses/cm².

10. Procédé selon la revendication 1, dans lequel la densité à laquelle les cellules souches mésenchymateuses sont directement ensemencées comprend une densité d'environ 5 cellules souches mésenchymateuses/cm².

11. Procédé selon la revendication 1, dans lequel la densité à laquelle les cellules souches mésenchymateuses sont directement ensemencées comprend une densité d'environ 50 cellules souches mésenchymateuses/cm².

12. Procédé selon la revendication 7 ou la revendication 8, dans lequel l'étape du réensemencement des cellules souches mésenchymateuses inclut en outre le réensemencement des cellules souches mésenchymateuses avant qu'elles atteignent la confluence.
